# EUROPEAN PATENT SPECIFICATION

(11) **EP 0 481 820 B1**
(45) Date of publication and mention of the grant of the patent: **18.12.1996**
(21) Application number: 91309665.7
(22) Date of filing: 18.10.1991
(51) Int. Cl.: C12M 3/04

(54) **Continuous high-density cell culture system**
Anlage zur kontinuierlichen Züchtung von Zellen mit hoher Dichte
Système pour la culture en continu de cellules à haute densité

(30) Priority: 18.10.1990 US 600195
(43) Date of publication of application: 22.04.1992
(73) Proprietor: COSTAR CORPORATION, Cambridge Massachusetts 02140 (US)
(72) Inventor: Berry, Eric Scott, Merrimack, New Hampshire 03054 (US); Butz, David, Westford, Massachusetts 01886 (US); Lawton, Lewis, Ossippee, New Hampshire (US); Szlosek, Paul, Kennebunk, Main 04043 (US)
(74) Representative: Jump, Timothy John Simon

(56) References cited:
- WO-A-90/01981
- WO-A-92/05243
- FR-A- 2 304 357
- US-A- 3 853 712
- US-A- 4 307 193
- PATENT ABSTRACTS OF JAPAN vol. 13, no. 29 (C-562)(3377) 23 January 1989

## Description

This invention relates to a method and apparatus for the culture of cells and more particularly relates to an improvement of the apparatus disclosed in co-pending international application WO-A-92/05243.

Conventionally, cells have been grown attached to glass or plastic roller bottles and flasks. This approach does not lend itself to high-density growth of cells or continuous cell culture, and requires large amounts of medium and space. Further, this approach is iabor intensive.

To achieve higher-density growth conditions, various attempts have been made to use arrangements of stacked plates, the surfaces of the stacked plates providing increased surface area for cell attachment and growth.

U.S. Patent No. 4,307,193 discloses a method for producing interferon from tissue culture cells using a propagating apparatus having a plurality of spaced-apart flat plates, which can be interconnected to allow medium and gas to pass between successive plates.

In spite of the various attempts, the cell-culturing devices of the prior art all have various drawbacks, including the need for excessive amounts of medium, the inability to provide a continuous flow of nutrients to all cell-growth surfaces, the need for labor-intensive monitoring and care of the growing cells and the inability to operate continuously.

An object of this invention is to provide a cell culture device in which cells may be grown to a high density relative to the amount of nutrient medium contained within the system.

Another object of this invention is to provide a cell culture assembly having a plurality of growth chambers for receiving medium flow substantially equally.

Another object of this invention is to provide a cell culture assembly having a plurality of growth chambers, which has reduced manufacturing costs as compared to the system shown in WO-A-92/05243, supra.

Yet another object of this invention is to provide a more effective seal for each of the chambers of the cell culture assembly shown in WO-A-92/05243, supra.

According to the present invention there is provided a tissue culture plate assembly comprising:
a plurality of plates stacked one upon the other with adjacent plates defining growth chambers between them, the plates having edges which are aligned with one another in the stack,
inlet and outlet conduits connected to the stack for channeling fluid to and from the chambers, wherein each of said plates has inlet and outlet ducts that communicate with the inlet and outlet conduits to direct fluid through the chambers,
and a frame enclosing the edges of all the plates, hermetically sealing the chambers and mechanically interlocking the staked plates together.

Further embodiments of the invention are as defined in the sub-claims.

In one aspect the invention may provide a cell culture assembly that allows for the automatic, continuous addition of nutrient medium and the removal of conditioned medium containing the products and waste formed by the cells.

In another aspect the invention may provide a cell culture assembly and system in which the cellular environmental condition may be continuously monitored, with any departures from the desired conditions being automatically corrected or alarmed.

In a further aspect the invention may be arranged to provide a continuous flow system to facilitate a desirable chemostatic environment for the cells and to facilitate optimal yield and harvesting ability for cell products such as biochemicals, vaccine virus, and pharmaceuticals.

The invention provides a continous cell culturing device having an array of growth chambers defining a very large surface area for high-density cell growth in a small volume. The device is constructed and arranged to permit directional flow through the growth chambers, the flow being continuous and capable of reaching all growth surfaces within each chamber. Adequate flow in each chamber is accomplished by providing each chamber with a fluid restriction port, these ports acting to control the flow and distribution of fluid into each of the growth chambers.

Preferably, the cell culturing device includes an array of cell growth chambers defined by the spaces between a plurality of stacked plates. An inlet conduit provides a source of nutrient medium to a manifold in fluid communication with the cell growth chambers via the fluid restriction ports. The sum of the cross-sectional areas of the narrowest region of the fluid restriction ports is less than the cross-sectional area of the inlet conduit, thereby ensuring a pressure drop across each of the fluid restriction ports and adequate flow of fluid through each growth chamber.

Preferably, the fluid restriction ports and growth chambers are constructed and arranged to promote the distribution and continuous directional flow of fluid medium to all growth surfaces with a minimum of turbulence. This may be accomplished by providing growth chambers that are essentially square boxes, with an inlet restriction port and an outlet restriction port at opposing corners of the box. The fluid restriction ports may have a constricted intermediate section, and the corners may have curved surfaces to promote fluid distribution and nonturbulent flow. Ribs may be provided between opposing faces of the plates to provide structural support and further to direct fluid flow.

The invention thus can provide a closed sterile system for the culture of cells and prevents both exposure of personnel to the cells and their products and the contamination of the culture from the outside environment. Growth conditions may be sensed automatically, with nutrients added and cell-products removed responsive to the sensed condition. The device can be supplied economically and can be manufactured in large scale production.

In a preferred embodiment of the present application, the stacked plates are sealed by a continuous frame that engages the edges of all the plates to seal the chambers. The frame can be molded about the plates as a single step and avoids the handling of the individual plates to separately seal the individual chambers. Because the assembly may have as many as twenty-five or fifty chambers formed by a corresponding number of plates stacked one upon another, this manufacturing procedure results in very substantial cost savings.

### Brief Description of the Drawings

FIG. 1 is a perspective view from the top right of the cell culture assembly of the invention;
FIG. 2 is a perspective cross-sectional view taken along line a-a of Fig. 1;
FIG. 3 is a perspective view from the top front of a single culture plate which may be used in forming the cell culture assembly of Fig. 1;
FIG. 4 is an enlarged view of a corner of the plate of Fig. 3;
FIG.5 is a perspective view of another embodiment of cell culture assembly constructed in accordance with this invention;
FIG. 6 is a fragmentary cross-section view of the assembly taken along the section line b-b in FIG. 5 ;
FIGS. 7 and 8 are plan views of the two faces of one of the many identical plates used in the assembly of FIGS. 5 and 6 ;
FIGS. 9-11 are fragmentary cross-sectional views of the plates taken along the section line c-c, d-d and e-e, respectively, in FIG. 5 ; and
FIGS. 12 and 13 are fragmentary cross-sectional views of the plan taken along the section lines f-f and g-g, respetively, in FIG.8.

### Detailed Description

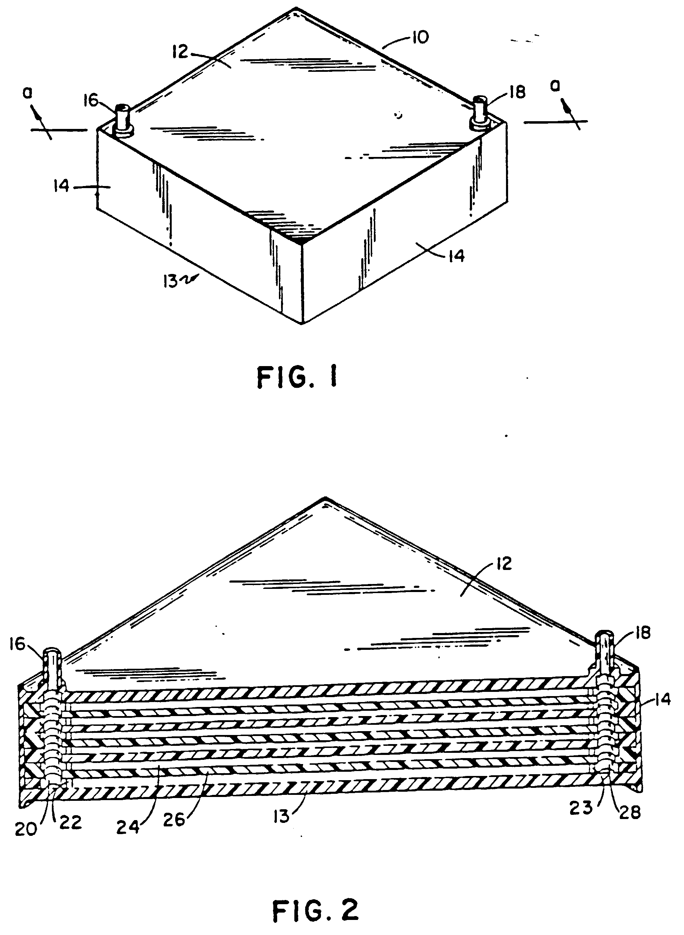

The cell culture device of the present invention embodiment includes an array of growth chambers enclosed within a vessel. The growth chambers provide a large surface area for cell growth. In exterior view (Fig. 1), the vessel 10 is a substantially square box with square, molded top 12 and base 13, and vertical molded sidewalls 14 sealed along the edges to provide a fluid-tight arrangement for housing the growth chambers. Extending from diagonally opposed corners of the top 12, are an inlet conduit 16 and an outlet conduit 18. The inlet conduit 16 provides fluid access to the interior space of the vessel 10, and the outlet conduit 18 provides fluid egress from the interior space of the vessel 10. The outlet conduit 18 may extend through the base 13, rather than through the top 12 as shown.

Referring to Fig. 2, the inlet conduit 16 is in axial alignment and in fluid communication with an inlet manifold 20 extending transversely through the interior space of the vessel 10 at the corner. Inlet restriction ports 22 provide fluid communication between the manifold 20 and a plurality of cell growth chambers 24 located within vessel 10, which chambers 24 are defined by an array of stacked plates 26. At the corner of the vessel opposite the inlet manifold 20 is an outlet manifold 28 in fluid communication with the outlet conduit 18. Each growth chamber 24 is in fluid communication with the outlet manifold 28 via an outlet fluid restriction port 23. The inlet and outlet manifold arrangements in accordance with one embodiment are mirror images of one another and, therefore, the flow characteristics will be the same whether the fluid medium is flowing from the inlet to the outlet ends, or vice versa.

The relative size of the restriction ports 22,23 and conduits 16,18 comprise one aspect of the invention. To ensure that each chamber receives a continuous and controlled flow of fluid medium, a pressure drop must be created across the flow restriction port controlling flow within each chamber. To achieve this, the sizes of the restriction ports controlling flow are selected such that the sum of the cross-sectional areas of the narrowest portion of these restriction ports is equal to or preferably less than the cross-sectional area of the inlet conduit. Preferably, the sum of these cross-sectional areas also is equal to or less than the cross-sectional area of the outlet conduit. When these conditions are present, no particular growth chamber will be favored due to its location, and all growth chambers will receive fluid medium based principally upon the particular size of the fluid restriction port controlling flow in that chamber. Preferably, the fluid restriction ports are uniformly dimensioned so that each growth chamber will receive fluid medium at an identical flow rate.

In the embodiment of FIGS. 2-4, there are flow restriction ports at both the inlet and the outlet end of the growth chambers. The smaller of the inlet and outlet restriction ports will control the flow through a particular growth chamber, without regard to its location at the inlet or outlet end. Thus, it is the sum of the cross-sectional areas of the smaller of the inlet and outlet restriction ports for each chamber that should be less than the cross-sectional area of the conduits. If a pair of inlet and outlet restriction ports are uniformly dimensioned and have the same cross-sectional area at their narrowest portion, then in calculating the sum of the cross-sectional areas as described above, only one cross-sectional area is included.

The plates 26 are stacked to form the growth chambers. Preferably, the plates are spaced 1 mm apart or greater. Although the plates may be spaced closer to one another, air bubbles tend tb become trapped between the plates and interrupt the even flow of medium when spaced less than 1 mm apart. The surfaces of the plates may be roughened, corrugated, convoluted or otherwise irregular to increase their surface area and the number of cells capable of growing on a given plate. If irregular, the 1 mm spacing between the plates should be between the facing peaks of irregular surfaces. The surfaces of the plates also may be surface treated in a variety of different ways to promote cell growth. Typical treatments include carboxyl group treatments, collagen treatments, fibronectin treatments or feeder cell layers.

The plates according to the preferred embodiment were molded from K-resin™⃝, a block co-polymer of polystyrene and butadiene, sold by Phillips Chemical Co., Bartlesville, OK, 74004. Suitable plate materials include styrenic materials or materials such as polymethyl pantene. However, the plate may be of virtually any material that is sufficiently strong, nontoxic, biocompatible, and otherwise suitable for tissue culture.

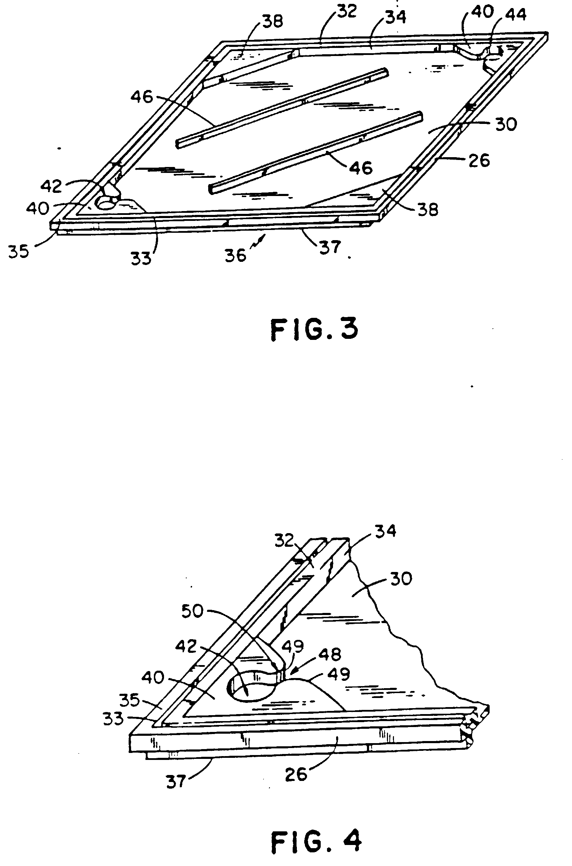

The plates of the embodiment of FIGS. 2-4 have a novel construction which facilitates flow distribution and manufacture. Each plate 26 has an upper surface 30 bounded by a peripheral wall 32 (Fig. 3). The inside facing surface 34 of wall 32 along with the plate upper surface 30 define the side walls and floor of a growth chamber 24. When the plates 26 are stacked in the assembled device, the top edge 35 of wall 32 mates and seals with the lower surface 36 of the adjacent plate to define a growth chamber, the ceiling of the chamber being defined by the lower surface 36 of the adjacent plate. To facilitate the mating arrangement of the stacked plates, the wall 32 is provided on its top edge 35 with a groove 33 for receiving a mating ridge 37 on the bottom surface of an adjacent plate. The mating ridge 37 and groove 33 align the stacked plates.

The four corners 38,38 and 40,40 of each plate 26 are solidly filled-in to a thickness equal to the height of the peripheral wall 32. The filled corners add support to the assembled structure. The filled corners further reduce the potential for fluid turbulence and "dead spots," as will be explained more fully below.

Each plate 26 has a bore at diagonally-opposed, filled corners 40. In the assembled condition, the bores 42,44 of the stacked plates 26 are axially aligned and form the inlet and outlet manifolds 20,28, respectively. A passage 48 extends from each bore 42,44 through the filled corners 40 to the interior space defined by the side walls and floor of each plate 26. These passages in the assembled vessel form the fluid restriction ports 22, 23 providing fluid access between the manifolds 20,28 and the growth chambers 24. Each passage has a floor defined by a portion of the upper surface 30 of the plate 26 and sidewalls 49 formed integrally as part of the corners 40. The height of each passage equals the height of the peripheral wall 32. The fluid restriction ports 22, 23 are formed when the flat bottom surface of an opposing plate 26 is stacked to seal the open upper extremity of the passage 48.

The particular shape of the fluid restriction port in the preferred embodiment includes a narrowed internal portion, as at constriction 50. The width of the flow restriction port 22 increases continuously and substantially from constriction 50 in a direction toward the growth chamber 24. This arrangement causes medium entering the growth chamber to flare-out and be evenly dispersed in a fan pattern into the growth chamber space, promoting nutrient supply and even fluid flow across the entire growth surface. This arrangement also reduces turbulence, especially at and around the restriction ports 22. In the embodiment shown, the width of the flow restriction port increases linearly while the height remaining constant. However, a port shaped as a second or third order venturi would provide for continuous, gradual change in velocity, thereby further promoting nonturbulent flow.

The nonturbulent and directional flow pattern is further facilitated by the filled corners 38 and by flow-guiding ribs 46. The corners, if not filled, would define areas remote from the direction of flow, which in turn would result in turbulence and "dead spots," that is, areas within the growth chamber which do not receive a continuous and directional flow of fresh media so as to cause cell death. The filled corners provide a baffle which acts to channel flow continuously in the direction of the outlet ports. The ribs 46 provide a dual function. They serve to provide structural support to each plate maintaining the plates at the proper spacing, even when draining the device under a vacuum, and also may be positioned to channel flow continuously in a direction toward the outlet ports.

To assemble the device, the plates must be stacked and secured to one another in a manner to hermetically seal the peripheral walls and corners of the plates to one another. In this embodiment each plate is welded to an adjacent plate by ultrasonic welding. However, any method of fabrication such as solvent welding, RF welding, potting, glue or even gaskets would be acceptable, so long as the final product has sufficient strength and is nontoxic to cultured cells.

In operation, the device first is seeded with cells. Then, fluid media is supplied to the attached cells as follows. Fluid medium is introduced into inlet manifold 20 via inlet conduit 16. The fluid medium then passes through the various flow restriction ports 22 into the associated growth chambers 24. Because of the overall construction of the flow restriction ports and the growth chambers, flow is distributed continuously and thoroughly to all surfaces of the growth chamber 24, the fluid medium always moving generally in a direction toward the outlet flow restrictors 23. The medium then passes through the outlet flow restrictors 23, into the outlet manifold 28 and then out of the device via the outlet conduit 18.

In the embodiments illustrated, only one pair of restriction ports per growth chamber has been described. It is, of course, possible to have many flow restrictors on the inlet or the outlet side. One inlet and one outlet restriction port may be preferred as they facilitate manufacture, lessen turbulence, and reduce the lack of cell growth normally found at and very close to the flow restriction ports.

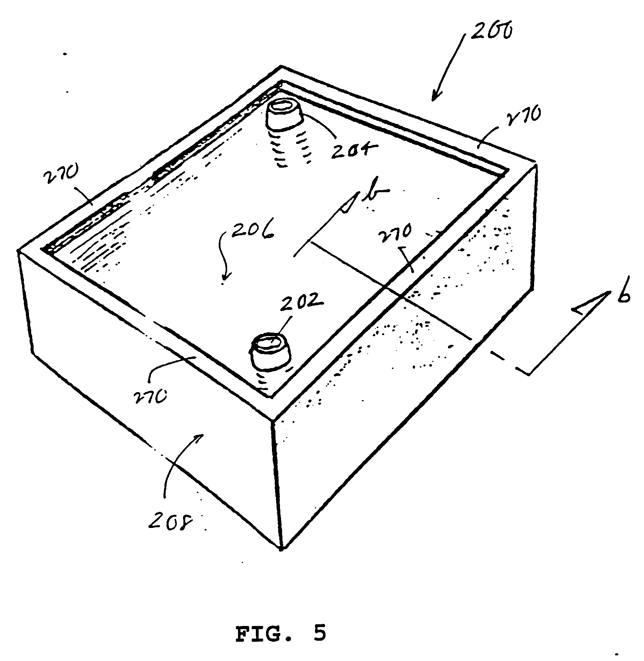

It further will be understood by one of ordinary skill in the art that it is possible to have flow restriction ports on the inlet side only. A vessel of such construction may be preferred for applications requiring the recovery of cells from the vessel, as conventional methods for removing cells from culture vessels tend to yield cell clumps which may clog a constricted outlet. It also is possible to have restriction ports on the outlet side only. The embodiment of FIGS. 5-13 includes only the inlet constrictions ports.

It further is preferred that the sum of the cross-sectional areas of the flow restriction ports be less than or equal to the cross-sectional area of the outlet conduit. Otherwise, the outlet conduit will set up a back-pressure which may result in preferential flow through one or more growth chambers.

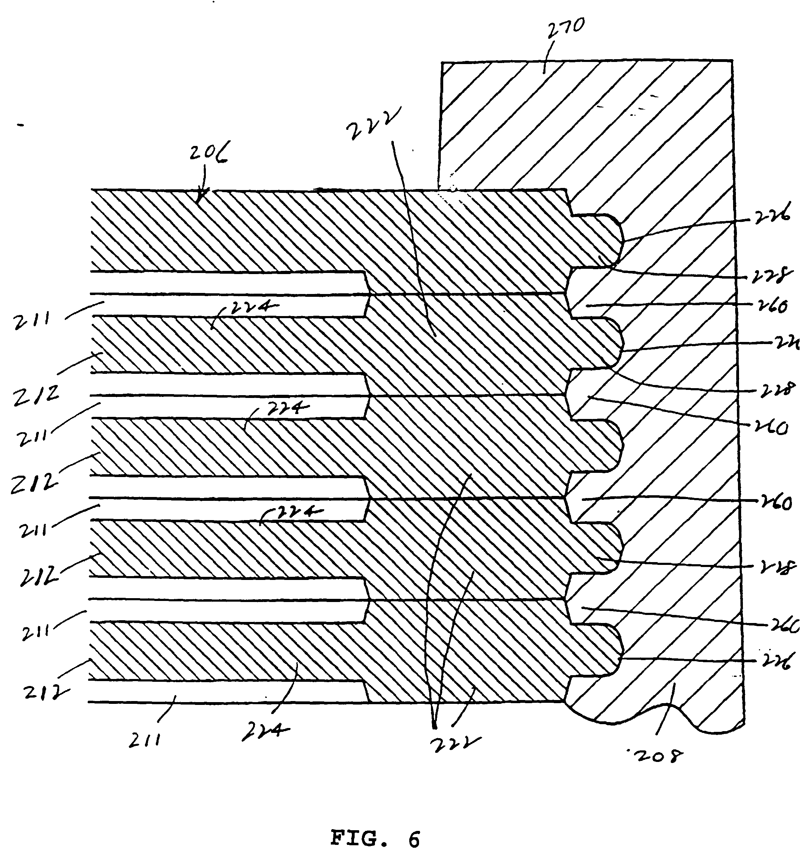

In FIG. 5 another embodiment of a cell culturing assembly is shown that functionally is the same as the embodiment shown in FIG. 1. The assembly differs from that of FIG. 1 in the manner in which the array of plates are held together. The assembly 200 shown in FIG. 5 includes inlet and outlet conduits 202 and 204 respectively, a stack of plates 206 and a frame 208 that holds the stack of plates together and hermetically seals the stack of chambers formed between them. In FIG. 6 , only a few of the stack of plates are shown sealed within the frame 208, but it should be appreciated that as many as 25 or 50 plates may be stacked one upon another to form a corresponding number of chambers.

In FIGS. 7-13 , one plate 212 that may be used in the stack of FIG. 6 is shown but it is to be understood that other plates such as shown in FIGS. 3 and 4 may be used instead. The frame 208 is not limited to just one plate configuration. At opposed corners 214 and 216 inlet and outlet ports 218 and 220 are provided which correspond to the ports 42 an 44 in the embodiment of FIGS. 3 and 4. A thickened rim 222 extends about the four sides of the plate from each surface thereof and surrounds the thin major portion 224 of the plate (see FIGS. 7-9. The rim 222 is spaced inwardly from the outer edge 226 of the plate to define a tongue 228 whose function is described below.

The two surfaces of the plates are substantially identical except for aligning risks in two corners as described below.

The ports 218 and 220 extend through the thin major portion 224 of the plate, and a pair of V-shaped ribs 230 and 232 disposed on each side of port 218 define a restricted flow path 234 between the adjacent apexes of the V-shaped ribs 230 and 232. No restricted passage is formed adjacent the outlet port 220 that corresponds to the restricted passage 234.

At the other pair of opposed corners 240 and 242, diagonal ribs 244 and 246, respectively, connect adjacent portions of the rim 222 and define baffles in the chambers so as to eliminate the so called "dead spots" in the corners of the chambers remote from the flow path between the inlet and outlet ducts 218 and 220. These ribs essentially perform the same function as the fill at the opposed corners in FIGS. 3 and 4. In FIGS. 7 and 8, the top and bottom surfaces of the plate 212 are shown, and it is evident from them that the two surfaces of the plates as thus described are essentially identical. Therefore, when two identical plates are placed face to face, the rims 222 stack one upon another as shown in FIG. 6 and the ribs 230, 232, 244 and 246 engage one another to form walls that in part define the growing chambers between the plates. Similarly, the transversely extending ribs 248 that correspond to the ribs 46 in the embodiment of FIGS. 3 and 4 register with one another to direct flow through the chamber.

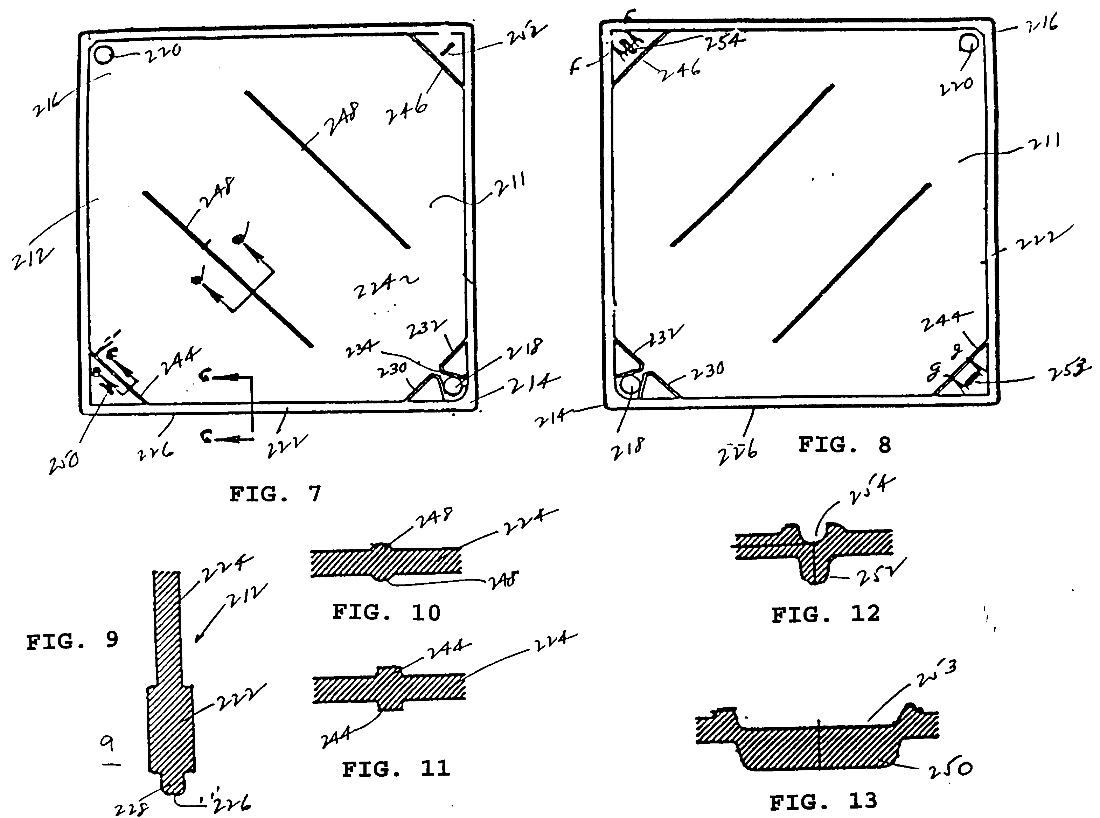

In the opposed corners 240 and 242 of the plate 212 stacking ribs 250 and 252 are provided on one face of the plate while corresponding troughs 253 and 254 are formed on the opposite face thereof. Cross-sections of the ribs and troughs are shown in FIGS. 12 and 13. It will be noted in the cross-sectional views that the troughs are slightly longer than the ribs so as to assist in centering the plates one upon another. It will be appreciated that because the ribs 250 and 252 and troughs 253 and 254 at the opposite corners are disposed at an angle to one another, the troughs and ribs prevent the plates from being stacked in any way but with inlet and outlet ports 218 and 220 of each plate properly aligned at the same corners of the stack. Moreover, the ribs and troughs will serve to hold several plates in the precise alignment with one another before the frame 208 is molded about them.

In the previously described embodiments no special frame is illustrated for holding the array of plates together and hermetically sealing the chambers, and various methods are suggested for assembling the array of plates, such as ultrasonic welding, solvent welding, potting etc. In this embodiment, the array of plates are held together by frame 208. Very substantial savings in manufacturing costs are derived from this system as well as a better seal for each chamber 211 and an aesthetically superior product is achieved as compared to the vessel manufactured by the other techniques described. The frame is molded by placing the stack of plates in a tool cavity and injecting the styrenic material from which the frame is made into the cavity to form it. It will be noted in FIGS. 9 and 10 that the frame material completely encloses the tongues 228 and.fills the cavity 260 between adjacent tongues to the interface of the adjacent rims 222. The frame totally encloses the edges of each of the identical plates in the stack and extends around the outer edges of the end plates in the stack. This technique which is commonly called insert molding not only bonds the frame 208 to the plates but also forms a mechanical interlock between the plates may be so that the assembly cannot fall apart. The principle advantage of this assembly is that all of the individual plates may be secured together in a single molding step as opposed to the separate steps required by the other techniques to attach each of the individual plates together. The importance of this feature will be particularly appreciated when it is realized that as many as 25 or 50 plates may be stacked in a single assembly, and the perimeter of each plate may be approximately 36 inches. Therefore, in a stack of 50 plates, approximately 150 linear feet of seal must be provided about the chambers. Furthermore, in many applications, so long as the frame 208 holds the stack together and seals with the end plates, a failure of a seal between adjacent inner plates will not be catastrophic for the frame will prevent the loss of any fluid from the system and the only exit for the fluid is the outlet conduit 204.

In FIG. 6 , it will be noted that the outer face of the topmost plate in the stack does not have the same configuration as the inner face. However, the inner face is identical to the lower faces of the plates below to form a chamber 211. Similarly, only the top face of the lower plate (not shown) is configured to form a chamber 211.

While polystryene has been suggested as one material from which the frame may be made, it will be appreciated that other materials may be used as well so long as they are compatible with the function performed by the assembly. For example, the frame could be injection molded from a rubber-like styrenic material which would provide physical protection for the assembly if it is dropped or otherwise subject to a sharp impact.

As suggested above, the plates may typically be approximately 58 cm² (9 inches square), and a very substantial number of plates may be used in a single assembly to form as many as 25 or 50 chambers. In such a construction, the wall thickness of the frame measured to the outer edges of the tongues may be approximately 0.238 cm (3/32 inch) and the outer flange 270 of the frame over the outer surfaces of the end plates as shown in FIG. 10 may be approximately 0.476 cm² (3/16 inch). Because the frame is continuous, it permanently and securely bonds the stack together so that the plates cannot accidentally separate nor can any leakage occur from the system so long as the bonds between the frame and the outer surfaces of the end plates are secure.

## Claims

1. A tissue culture plate assembly (200) comprising:
a plurality of plates (212) stacked one upon the other with adjacent plates defining growth chambers (211) between them, the plates having edges (226) which are aligned with one another in the stack (206),
inlet and outlet conduits (202 and 204) connected to the stack for channeling fluid to and from the chambers (211) wherein each of said plates (212) has inlet and outlet ducts (218 and 220) that communicate with the inlet and outlet conduits (202 and 204) to direct fluid through the chambers (211),
and a frame (208) enclosing the edges of all the plates, hermetically sealing the chambers (211) and mechanically interlocking the stacked plates (212) together.

2. A tissue culture plate assembly (200) as defined in claim 1 wherein the frame (208) is molded as a unitary structure about all the edges of all the plates (212).

3. A tissue culture plate assembly (200) as defined in claims 1 or 2 wherein the plates (212) in the stack are identical with one another.

4. A tissue culture plate assembly (200) as defined in any of claims 1-3 wherein each of the plates (212) has a rim (222) adjacent the edge (226) and which extends about the periphery thereof, the rims (222) of adjacent plates being stacked one on the other and providing side walls for the chambers (211).

5. A tissue culture plate assembly (200) as defined in any of claims 1-4 wherein tongues (228) are formed in the edges (226) of the plates with the tongues (228) of adjacent plates (212) in the stack (206) forming cavities (260) between them, said frame (208) filling the cavities between the tongues (228) and preferably bonding the plates (212) together.

6. A tissue culture plate assembly (200) as defined in any of the preceding claims further including restricted passages (234) provided in each of said plates and connecting the inlet duct (218) to the chambers (211) on each side of each of said plates (212).

7. A tissue culture plate assembly (200) as defined in claim 6 further including ribs (230 and 232) provided in a plate (212) adjacent the inlet duct (218) to define the restricted passages (234).

8. A tissue culture plate assembly (200) as defined in any of claims 1-7 further including stacking ribs (250 and 252) and grooves (253 and 254) on opposite faces of each of the identical plates (212) for aligning the plates (212) with one another in a preselected relationship.

9. A tissue culture plate assembly (200) as defined in any of the preceding claims wherein the chambers (211) are at least 1 mm in height.

10. A tissue culture plate assembly (200) as defined in any of the preceding claims wherein the plates (211) are substantially square.

11. A tissue culture plate assembly (200) as defined in any of the preceding claims wherein support ribs (230, 232, 244 and 246) separate adjacent plates (212) in the chamber (211).

12. A tissue culture plate assembly (200) as defined in claim 1 or claim 2 wherein protrusions (228) are formed in the edges (226) of the plates (212) with the protrusions (228) of adjacent plates (212)in the stack (206) forming cavities (260) between them, said frame (208) filling the cavities (260) between the protrusions (228) and bonding the plates (212) together.

13. A tissue culture plate assembly (200) as defined in any of the preceding claims wherein the frame (208) contacts all of the edges (226) of each plate (212).

14. A tissue culture plate assembly (200) as defined in any of the preceding claims comprising at least 25 plates (212).

## Patentansprüche

1. Montagegruppe von Plättchen zur Züchtung von Gewebe (200), bestehend aus einer Vielzahl von übereinander gestapelten Plättchen (212), wobei angrenzende Plättchen zwischen sich Wachstumskammern (211) bestimmen und die Plättchen Ränder (226) aufweisen, die miteinander in einem Stapel (206) ausgerichtet sind, Zu- und Ableitungen (202,206), die mit dem Stapel zur Kanalisierung von Flüssigkeit zu und von den Kammern (211) verbunden sind, wobei jedes der besagten Plättchen (212) Zu- und Ableitungsröhren (218,220) aufweist, die in Verbindung mit den Zu- und Ableitungen (202,204) stehen, um die Flüssigkeit durch die Kammern zu leiten sowie einem Rahmen (208), der die Ecken aller Plättchen umschließt und die Kammern (211) hermetisch abdichtet sowie die gestapelten Plättchen (212) mechanisch miteinander verbindet.

2. Montagegruppe von Plättchen zur Züchtung von Gewebe (200) nach Anspruch 1, dadurch gekennzeichnet, daß der Rahmen (208) als einheitliche Struktur um alle Ecken aller Plättchen (212) geformt ist.

3. Montagegruppe von Plättchen zur Züchtung von Gewebe (200) nach Anspruch 1 oder 2, dadurch gekennzeichnet, daß die Plättchen (212) in dem Stapel identisch sind.

4. Montagegruppe von Plättchen zur Züchtung von Gewebe (200) nach einem der Ansprüche 1 bis 3, dadurch gekennzeichnet, daß jedes der Plättchen (212) einen an die Kante (226) angrenzende Rand (222) aufweist, wobei die Ränder (222) angrenzender und sich über deren Peripherie heraus erstreckende Plättchen übereinander gestapelt werden und die Seitenwände der Kammern (211) ausbilden.

5. Montagegruppe von Plättchen zur Züchtung von Gewebe (200) nach einem der Ansprüche 1 bis 4, dadurch gekennzeichnet, daß die Zungen (228) an den Kanten (226) der Plättchen ausgebildet werden, wobei die Zungen (228) der angrenzenden Plättchen (212) im Stapel (206) zwischeneinander Hohlräume (260) bilden und besagter Rahmen (208) die Höhlungen zwischen den Zungen (228) füllt und die Plättchen (212) vorzugsweise zusammenkittet.

6. Montagegruppe von Plättchen zur Züchtung von Gewebe (200) nach einem der Ansprüche 1 bis 5, zusätzlich dadurch gekennzeichnet, daß verengte Durchgänge (234) in jedem der besagten Plättchen vorgesehen sind, die die Zuleitung (218) auf jeder Seite jedes der besagten Plättchen (212) mit den Kammern (211) verbinden.

7. Montagegruppe von Plättchen zur Züchtung von Gewebe (200) nach Anspruch 6, zusätzlich dadurch gekennzeichnet, daß in einem an die Zuleitung (218) angrenzenden Plättchen (212) Leisten (230,232) angeordnet sind, die die verengten Durchgänge (234) bilden.

8. Montagegruppe von Plättchen zur Züchtung von Gewebe (200) nach einem der Ansprüche 1 bis 7, dadurch gekennzeichnet, daß Stapelleisten (250,252) und Rillen (253,254) auf gegenüberliegenden Seiten jedes der identischen Plättchen (212) ausgebildet sind, die die Plättchen (212) zueinander in einer vorher gewählten Verbindung ausrichten.

9. Montagegruppe von Plättchen zur Züchtung von Gewebe (200) nach einem der Ansprüche 1 bis 8, dadurch gekennzeichnet, daß die Kammern (211) mindestens 1 mm Höhe aufweisen.

10. Montagegruppe von Plättchen zur Züchtung von Gewebe (200) nach einem der Ansprüche 1 bis 9, dadurch gekennzeichnet, daß die Plättchen im wesentlichen quadratisch sind.

11. Montagegruppe von Plättchen zur Züchtung von Gewebe (200) nach einem der Ansprüche 1 bis 10, dadurch gekennzeichnet, daß Unterstützungsleisten (230,232,244,246) gegeneinandergrenzende Plättchen (212) in der Kammer (211) trennen.

12. Montagegruppe von Plättchen zur Züchtung von Gewebe (200) nach einem der Ansprüche 1 oder 2, dadurch gekennzeichnet, daß an den Kanten (226) der Plättchen (212) Vorwölbungen (228) ausgebildet sind, wobei die Vorwölbungen (228) benachbarter Plättchen (212) im Stapel (206) Aushöhlungen (260) zwischeneinander bilden und besagter Rahmen (208) die Aushöhlungen (260) zwischen den Vorwölbungen (228) füllt und die Plättchen (212) verkittet.

13. Montagegruppe von Plättchen zur Züchtung von Gewebe (200) nach einem der Ansprüche 1 bis 12, dadurch gekennzeichnet, daß der Rahmen (208) mit sämtlichen Ecken (226) jedes der Plättchen (212) verbunden ist.

14. Montagegruppe von Plättchen zur Züchtung von Gewebe (200) nach einem der Ansprüche 1 bis 13, dadurch gekennzeichnet, daß sie aus mindestens fünfundzwanzig Plättchen (212) besteht.

## Revendications

1. Ensemble de plaques de culture de tissus (200) comprenant :
une pluralité de plaques (212) empilées les unes sur les autres avec des plaques adjacentes définissant des chambres de croissance (211) entre elles, les plaques présentant des bords (226) qui sont alignés les uns par rapport aux autres dans la pile (206),
des tubes d'entrée et de sortie (202 et 204) reliés à la pile afin de canaliser le fluide en direction de et à partir des chambres (211) dans lesquelles chacune desdites plaques (212) présente des conduits d'entrée et de sortie (218 et 220) qui communiquent avec les tubes d'entrée et de sortie (202 et 204) afin de diriger le fluide à travers les chambres (211),
et un châssis (208) qui enclôt les bords de toutes les plaques, scellant les chambres (211) de façon étanche et verrouillant ensemble, d'une manière mécanique, les plaques empilées (212).

2. Ensemble de plaques de culture de tissus (200) défini dans la revendication 1, dans lequel le châssis (208) est moulé comme structure en un seul bloc autour de tous les bords de toutes les plaques (212).

3. Ensemble de plaques de culture de tissus (200) défini dans les revendications 1 ou 2, dans lequel les plaques (212) de la pile sont identiques les unes aux autres.

4. Ensemble de plaques de culture de tissus (200) défini dans l'une quelconque des revendications 1 à 3, dans lequel chacune des plaques (212) présente un rebord (222) adjacent au bord (226) qui s'étend autour de la périphérie de celui-ci, les rebords (222) de plaques adjacentes étant empilés les uns sur les autres et formant des parois latérales pour les chambres (211).

5. Ensemble de plaques de culture de tissus (200) défini dans l'une quelconque des revendications 1 à 4, dans lequel des languettes (228) sont formées dans les bords (226) des plaques, les languettes (228) des plaques adjacentes (212) de la pile (206) formant des cavités (260) entre celles-ci, ledit châssis (208) remplissant les cavités entre les languettes (228) et liant, de préférence, les plaques (212) ensemble.

6. Ensemble de plaques de culture de tissus (200) défini dans l'une quelconque des revendications précédentes, comportant en outre des passages (234) limités formés dans chacune desdites plaques et reliant le conduit d'entrée (218) aux chambres (211) de chaque côté de chacune desdites plaques (212).

7. Ensemble de plaques de culture de tissus (200) défini dans la revendication 6, comportant en outre des nervures (230 et 232) formées dans une plaque (212) adjacente au conduit d'entrée (218) afin de définir les passages limités (234)

8. Ensemble de plaques de culture de tissus (200) défini dans l'une quelconque des revendications 1 à 7, comportant en outre des nervures d'empilage (250 et 252) et des rainures (253 et 254) sur des faces opposées de chacune des plaques identiques (212) afin d'aligner les plaques (212) les unes avec les autres dans une relation choisie préalablement.

9. Ensemble de plaques de culture de tissus (200) défini dans l'une quelconque des revendications précédentes, dans lequel les chambres (211) ont au moins 1 mm d'épaisseur.

10. Ensemble de plaques de culture de tissus (200) défini dans l'une quelconque des revendications précédentes, dans lequel les plaques (211) sont sensiblement carrées.

11. Ensemble de plaques de culture de tissus (200) défini dans l'une quelconque des revendications précédentes, dans lequel des nervures de support (230, 232, 244 et 246) séparent des plaques adjacentes (212) dans la chambre (211).

12. Ensemble de plaques de culture de tissus (200) défini dans les revendications 1 ou 2, dans lequel des saillies (228) sont formées dans les bords (226) des plaques (212), les saillies (228) des plaques adjacentes (212) dans la pile (206) formant des cavités (260) entre celles-ci, ledit châssis (208) remplissant les cavités (260) entre les saillies (228) et liant les plaques (212) ensemble.

13. Ensemble de plaques de culture de tissus (200) défini dans l'une quelconque des revendications précédentes, dans lequel le châssis (208) est en contact avec tous les bords (226) de chaque plaque (212).

14. Ensemble de plaques de culture de tissus (200) défini dans l'une quelconque des revendications précédentes, comprenant au moins 25 plaques (212).
